# EUROPEAN PATENT APPLICATION

(11) **EP 0 711 503 A2**
(43) Date of publication of application: **15.05.1996**
(21) Application number: 95308006.6
(22) Date of filing: 09.11.1995
(51) Int. Cl.: A23C 9/152, A23C 11/04, A23C 19/055, A23C 9/13, A61K 31/20

(54) **Milk fortified with GLA and/or DGLA**

(30) Priority: 14.11.1994 GB 9422897; 13.12.1994 GB 9425101
(71) Applicant: SCOTIA HOLDINGS PLC, Guildford Surrey GU1 1BA (GB)
(72) Inventor: Horrobin, David Frederick, Dr., c/o Scotia, Guildford, Surrey GU1 1BA (GB)
(74) Representative: Sturt, Clifford Mark

(57) **Abstract**

A milk or milk-derived product, enriched or supplemented with GLA and/or DGLA, the milk or milk-derived product comprising 100mg to 30g/100ml preferably 150mg to 5g/100ml and very preferably 200mg to 2g per 100ml of GLA and/or DGLA and optionally further comprising fatty acids such as AA, SA, EPA, or DHA in amounts from 1mg to 30g/100ml.

## Description

Milk, primarily from cows, is one of the most abundantly used foods in the world. It has excellent nutrient qualities, providing calories and a wide range of essential vitamins and minerals including calcium. However, over the past forty years it has fallen progressively into disfavour because of the large amount of fat, and particularly saturated fat, which it contains. Because of this, semi-skimmed, skimmed and powdered milks have become increasingly popular, and the consumption of milk overall has fallen progressively.

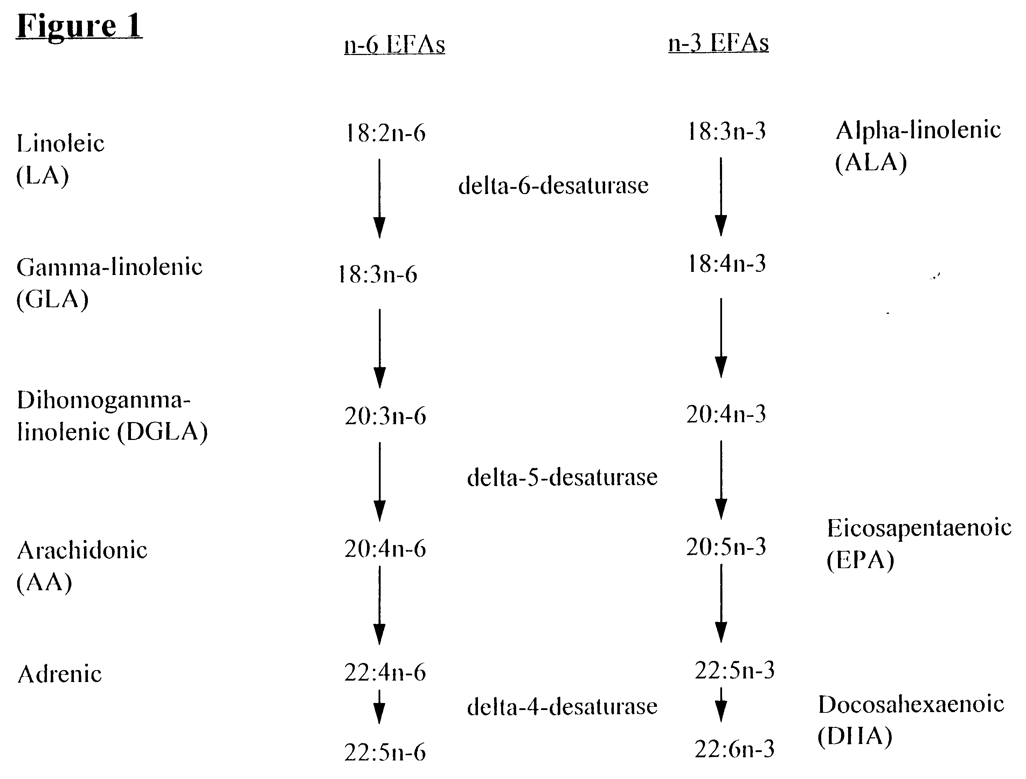

However, it has been argued that saturated fats themselves are not harmful but problems arise because of their diluting effect on certain key essential nutrients known as the essential fatty acids. There are twelve of these as set out in Table 1. Most diets contain reasonably substantial amounts of the parent fatty acids, linoleic (LA), and alphalinolenic acid (ALA). However, in order to perform most of their functions within the body, LA and ALA must first undergo 6-desaturation, by the rate liming enzyme, delta-6-desaturase and then be converted to the further fatty acids shown in the figure. Among these, dihomogammalinolenic acid (DGLA) and arachidonic acid (AA) of the omega-6 series and eicosapentaenoic acid (EPA) and docosahexaenoic acid (DHA) of the omega-3 series, are particularly important. Gamma-linolenic acid (GLA) and stearidonic acid (SA) are also important because they are beyond the rate-limiting step and can be converted to the other fatty acids relatively easily. GLA and DGLA have a very wide range of desirable effects and are valuable in the promotion of human health.

In humans 6-desaturation seems particularly slow. Only 10-20% of dietary ALA and only 1-3% of dietary LA are converted to their metabolites. This explains why human milk, unlike cows' milk, contains substantial amounts of DGLA, GLA, AA, EPA and DHA. Human babies need these fatty acids to be provided because they cannot make them rapidly enough themselves. Until very recently, artificial infant formulae contained either only linoleic acid or LA + ALA but did not contain further metabolites. Now infant formulae have been made containing GLA, AA and DHA and several patents have been filed in this field, for example EP-0140805B and US-4670285. None has involved a GLA concentration of more than 100mg/100ml.

However, it is not only infants who benefit from the metabolites of the parent fatty acids. The n-6 EFAs, GLA and DGLA and AA, are of value in many situations including skin disorders such as eczema and acne; diabetes and its complications; hypertension and other cardiovascular disorders including coronary and peripheral vascular disease; inflammatory disorders such as rheumatoid and osteo-arthritis, systemic lupus erythematosus, Crohn's disease, ulcerative colitis and many others; respiratory disorders such as asthma; psychiatric disorders such as schizophrenia and alcoholism; gastrointestinal disorders such as peptic ulceration; some cancers; osteoporosis; prostatic disorders; and impaired renal function. Omega-3 fatty acids such as EPA and DHA may also be of value. Many of the above disorders may benefit from co-administration of EFAs from both the ω-6 and ω-3 series. Much attention has been paid to the ω-3 series but much less attention has been directed to the ω-6 series. It is the inventor's contention that GLA and DGLA are of particular value in human health.

Milk, whether full cream, skimmed or partly skimmed is a convenient vehicle for the administration of fatty acids such as GLA and DGLA. Optionally other fatty acids such as AA, SA, EPA and DHA may also be added. Cow's milk is suitable for such use, but so is other milk for example milk from goats, sheep or camels. At low concentrations, fatty acids in glyceride, ester, salt, free fatty acid or phospholipid forms readily form stable emulsions when shaken up with milk. At higher concentrations the addition of emulsifiers may be of value in improving the formulation. Any acceptable emulsifier may be used, but we have found that galactolipids from plant sources and purified milk and egg sphingolipids are of particular value in producing stable emulsions. Because the fatty acids are easily oxidised, it is also desirable to add an appropriate anti-oxidant. We have found that while a number of commonly used antioxidants are of value, the ascorbate/ phosphorylated mono- or di-acyl glyceride system described in earlier patent applications (such as EP-0577305A) is of particular value.

GLA may be obtained as a glyceride or as various fully or partially purified derivatives from many sources including the oils of evening primrose, borage, blackcurrant, and various fungi and algae including Mucor, Rhizopus and Spirulina. DGLA can be synthesised from GLA or isolated from various animal tissues, particularly liver, kidneys, adrenals or gonads. AA can be isolated from the same animal tissues or from egg yolk or obtained from various fungal and algal oils. SA is found in a number of vegetable oils including blackcurrant and blue bur (Lappula Squarrosa) oils and in fungal and algal oils. EPA and DHA are found in marine oils and various algal and fungal oils. All the fatty acids can be chemically synthesised although at present the methods are difficult and expensive.

The invention is particularly directed to the addition of GLA and/or DGLA in therapeutically useful quantities to milks from cows, goats or sheep, whether full milk, skimmed or semi-skimmed and also to artificial milks such as soy milk or other milk substitute. Milk products such as yoghurts, creams, cheeses, butters, fromage frais, and any other fermented or unfermented milk products may also be used. The aim is to achieve a useful therapeutic dose of GLA or DGLA in a portion of milk or other product which might reasonably be consumed by one individual at a single time. The lowest level of therapeutically useful GLA or DGLA is likely to be 100mg/100ml in milks and 100 mg/50ml in other products.

Either fatty acid in any appropriate form may be incorporated into a cows milk emulsion at levels of from 100mg to 30g/100ml, preferably 150mg to 5g/100ml, and very preferably 200mg to 2g/100ml. Glycerides, phospholipids and esters are the preferred forms because they have relatively bland tastes. However, any other derivative which can be made palatable and which deliver the GLA or DGLA in an assimilable form are acceptable. It is difficult to prepare emulsions in which the total fatty acid concentration is above about 30g/100ml. Either whole, skimmed or partially skimmed milks may be used to make emulsions. The milks may be flavoured and/or sweetened in any appropriate way using any appropriate flavours or sweeteners. The emulsions may also be enriched with other nutrients such as calcium, vitamin C, or any other water soluble or fat soluble nutrient substance. The emulsions may also be used as vehicles for natural or synthetic products with therapeutic potential. The emulsions may be presented in unsterilised, pasteurised or sterilised forms as required. The outer packaging may be anything appropriate for milk.

### Aspects of the invention

1. Milk enriched or supplemented with 100mg to 30g/100ml, preferably 150mg to 5g/100ml and very preferably 200mg to 2g/100ml of GLA and/or DGLA. Other fatty acids such as AA or SA or EPA or DHA may be optionally added in amounts from lmg to 30g/100ml, preferably 150mg to 5g/100ml and very preferably 200mg to 2g/100ml.
2. Milk enriched or supplemented with GLA and/or DGLA as in (1) above, with or without additional calcium and optionally with the addition of other fatty acid(s), particularly EPA, for the enhancement of calcium absorption, of calcium balance and the treatment and prevention of osteoporosis. The calcium may be added in any appropriate assimilable form so that 50mg to 2000mg of additional calcium are added to 100ml. Appropriate sources of calcium include, but are not limited to, the calcium salt of an essential fatty acid, calcium chloride, calcium gluconate, calcium lactate, calcium carbonate, calcium phosphate, calcium levulinate and calcium glubionate.
3. Milk enriched or supplemented with GLA and/or DGLA as in (1) for the prevention and/or treatment of any of the disorders listed herein.
4. Milk enriched or supplemented with GLA and/or DGLA as in (1) for the treatment and prevention of atopic eczema.
5. Milk enriched or supplemented with GLA and/or DGLA as in (1) optionally with EPA or DHA, for the prevention and treatment of rheumatoid or osteo-arthritis.
6. Milk enriched or supplemented with GLA and/or DGLA as in (1), optionally with EPA, DHA or AA for the treatment of schizophrenia or alcoholism.
7. Milk enriched or supplemented with GLA and/or DGLA as in (1), optionally with AA, EPA or DHA, for the prevention and treatment of hypertension, coronary artery disease or peripheral vascular disease.
8. Milk enriched or supplemented with GLA and/or DGLA as in (1), optionally with other fatty acids or nutrients such as ascorbic acid or ascorbate in amounts from 10mg to 2000mg per 100ml for the treatment or prevention of asthma and of diabetes and its complications.
9. Milk enriched or supplemented with GLA and/or DGLA as in (1), optionally with other fatty acids and other nutrients such as zinc in amounts from 1mg to 800mg/100ml for the treatment and prevention of prostatic diseases, including prostatic hypertrophy.
10. Milk enriched or supplemented with GLA and/or DGLA as in (1), optionally with other fatty acids or other nutrients such as ascorbic acid or ascorbate in amounts from 10mg to 2000mg/100ml, for the supportive nutrition of patients with cancer.

There are further many foods derived from milk which are also suitable for modification by the addition of the essential fatty acids listed in this application. These foods include yoghurt, sour milk, buttermilk, whey, cream, fromage frais, cheese, butter and other milk-derived products. Artificial milks such as soy milk or other milk substitute are also appropriate for the addition of the relevant fatty acids.

Thus, further aspects of the invention are products as in paragraphs 1 to 10 above wherein the milk is in the form of, or replaced by, milk derivatives such as yoghurt, sour milk, buttermilk, whey, cream, fromage frais, cheese, butter and other milk-derived products or artificial milks such as soy milk or other milk substitute. In these instances the stated amounts of fatty acids may be added per 50 ml of any of the afore-mentioned milk derivatives or artifical milks.

The milks may be prepared by any method known to those skilled in the art. However, one convenient method which is easily applied in practice is to take the GLA or DGLA containing oil, add to it 0.1 - 5.0% of an appropriate emulsifier, which could be a phospholipid (such as phospholipid derived from eggs or from plants such as soya beans) or a synthetic emulsifier, but is preferably a plant galactolipid or a milk sphingolipid, and then to add this to the milk in equipment which permits intensive mixing.

### Examples of Formulations

All the formulations may be made using 0.1-5% of galactolipid, sphingolipid or phospholipid added to the fatty acid source as emulsifying agents in addition to the natural emulsifiers of the milk itself.
- 1.: Semi-skimmed milk containing a glyceride or phospholipid derived from any of the sources mentioned in the text which provides a level of 100-2000mg of GLA per 100ml.
- 2.: As (1), but with DGLA.
- 3.: As (1-2) but with two, three or four additional fatty acids selected from AA, SA EPA or DHA.
- 4-6: As (1-3) but prepared with additional calcium from any appropriate source but particularly the ones mentioned in the text to provide an extra 50-1000mg/100ml.
- 7-9: As (1-3) but prepared with additional ascorbic acid or ascorbate to provide an extra 10-2000mg/100ml.
- 10-12: As examples (1-3) but instead made using yoghurt, sour milk, buttermilk or whey, optionally with added calcium or ascorbic acid as in examples (4-6) and (7-9).
- 13-15: As examples (1-3) but instead made using artificial milks such as soy milk or other milk substitutes, optionally with added calcium or ascorbic acid as in examples (4-6) and (7-9).

The above examples may be formulated with any appropriate flavouring agent such as chocolate, vanilla, raspberry or strawberry, any appropriate colouring, any appropriate stabilising agent, and any appropriate emulsifying agent with the galactolipids at levels of 0.1%-5% of the fatty acids by weight being particularly valuable. Phospholipids or sphingolipids from any appropriate source such as soya beans, milk, eggs or other source may also be used.

### Examples of Galactolipid Emulsifiers

With regard to the galactolipids, two types of classes of lipids based on galactose are very common, that is mono-galactosyldiacylglycerol and digalactosyldiacylglycerol. Commonly used nomenclature and abbreviations are monogalactosyldiglyceride, MGDG, and digalactosyldiglyceride, DGDG. The general structure of said compounds are outlined below. 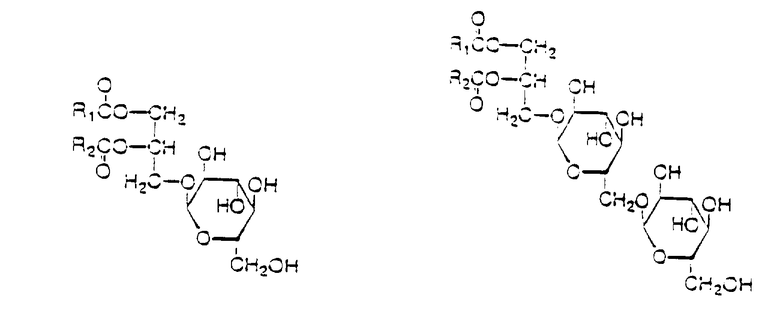 wherein R₁, R₂ = fatty acid residues

MGDG DGDG

To some extent one of the two fatty acid residues may be absent in the two lipids, which results in the corresponding monoacyl compounds, i.e. digalactosylmonoglyceride, DGMG, and monogalactosylmonoglyceride, MGMG. Higher homologues with additional galactose units may also occur in small amounts.

### Examples of Galactolipid Extraction

The glycosylglyceride material can be extracted from almost any kind of glycolipid-containing plant material. Preferred plant materials are seeds and kernels from grains and cereals, for instance, wheat, rye, oats, corn, rice, millet and sesame.

The following relates to a procedure for producing a glycolipid material from oat kernels.

200kg of oat kernels (Kungsörnen, Sweden) were grounded and extracted with 1000 l of 95% ethanol at 70°C for 3 h in an extraction tank under stirring. The slurry was centrifuged while still warm and separated from solid particles. The liquid fraction was evaporated at maximum 60°C which yielded about 10kg of extract in the form of a light brown oil.

The carbohydrates were removed in the following way:

10kg of the extract was carefully mixed with 10 l of hexane (hexane polymerisation grade, distillation range 65-70°C, Shell) and 50 l of 70% aqueous ethanol. The mixture separated into two phases over night. The lower, more polar phase containing the carbohydrates was discarded. The lipids are contained in the upper phase.

The upper phase, amounting to about 20 l, was applied to a stainless steel column of 30cm x 20cm (ID x h) having a volume of about 14 l and containing 6.25kg of silica gel (Matrex Silica Si, particle size 20-45 µm, pore diameter 60 Å, from Amicon Corporation, Danvers). The column was heated to 50°C and then washed with 30 l of a mixture of hexane:isopropanol, 90:10, in order to remove all triglycerides.

The glycolipid material was then eluted from the column with 20 l of a mixture of hexane:isopropanol, 60:40, giving a glycolipid fraction. Evaporation of the glycolipid fraction gave about 700g of a glycolipid material.

Analysis of the glycolipid material by HPLC indicated that the glycolipid material contains >70 area % DGDG. Two of the peaks, DGDG1 and DGDG2, respectively, represent two major classes of digalactosyldiglycerides having different acyl group structures. Some of the additional peaks have been identified as phospholipids, that is PE, phosphatidylethanolamine, PA, phosphatidic acid, PI, phosphatidylinositol and PC, phosphatidylcholine. The total amount of phospholipids is below 10 area %.

## Claims

1. A milk or milk-derived product, enriched or supplemented with GLA and/or DGLA, the milk or milk-derived product comprising 100mg to 30g, preferably 150mg to 5g and very preferably 200mg to 2g of GLA and/or DGLA per 100ml milk product or per 50 ml of milk-derived product and optionally further comprising fatty acids such as AA, SA, EPA, or DHA in amounts from lmg to 30g/100ml of milk or milk-derived product.

2. A milk or milk-derived product according to claim 1 further comprising additional calcium in any appropriate assimilable form providing 50mg to 2000mg of additional calcium per 100ml, and optionally further comprising other fatty acid(s) particularly, EPA.

3. A milk or milk-derived product according to claim 1 or claim 2 for the enhancement of calcium absorption, of calcium balance and the treatment and prevention of osteoporosis.

4. A milk or milk-derived product according to claim 1 for the prevention and/or treatment of any of the disorders listed herein.

5. A milk or milk-derived product according to claim 1 for the treatment and prevention of atopic eczema.

6. A milk or milk-derived product according to claim 1 optionally with EPA or DHA, for the prevention and treatment of rheumatoid or osteo-arthritis.

7. A milk or milk-derived product according to claim 1 optionally with EPA, DHA or AA for the treatment of schizophrenia or alcoholism.

8. A milk or milk-derived product according to claim 1 optionally with AA, EPA or DHA for the prevention or treatment of hypertension, coronary artery disease or peripheral vascular disease.

9. A milk or milk-derived product according to claim 1 optionally with other fatty acids or nutrients such as ascorbic acid or ascorbate in amounts from 10mg to 2000mg/100ml for the treatment or prevention of asthma and of diabetes and its complications.

10. A milk or milk-derived product according to claim 1 optionally with other fatty acids and other nutrients such as zinc in amounts from 1mg to 800mg/100ml for the treatment and prevention of prostatic diseases, including prostatic hypertrophy.

11. A milk or milk-derived product according to claim 1 optionally with other fatty acids and other nutrients such as ascorbic acid or ascorbate in amounts from 10mg to 2000mg/100ml, for the supportive nutrition of patients with cancer.

12. A milk or milk-derived product according to any preceding claim further comprising an appropriate emulsifier, such as a phospholipid or a synthetic emulsifier, preferably a plant galactolipid or a milk sphingolipid.

13. A milk or milk-derived product according to any preceding claim wherein said fatty acids and other nutrients are added to a milk or milk-derived product obtained from cows, goats, sheep or camels.

14. A milk or milk derived product according to any one of claims 1-12 wherein the milk is in the form of, or the milk-derived product is derived from, artificial soy milk or other milk substitute.

15. A milk or milk-derived product according to any preceding claim wherein the milk is in the form of, or the milk-derived product is selected from, yoghurt, sour milk, buttermilk, whey, cream, fromage frais, cheese, butter or other milk derivative.
